# EUROPÄISCHE PATENTSCHRIFT

(11) **EP 1 427 336 B1**
(45) Veröffentlichungstag und Bekanntmachung des Hinweises auf die Patenterteilung: **12.01.2005**
(21) Anmeldenummer: 02779333.0
(22) Anmeldetag: 11.09.2002
(51) Int. Cl.: A61B 5/07, A61B 5/00

(54) **INTRAKORPORALE SONDE ZUR ANALYSE ODER DIAGNOSE UND/ODER THERAPIE**
INTRACORPOREAL PROBE FOR ANALYSIS OR DIAGNOSIS AND/OR THERAPY IN HOLLOW ORGANS AND BODY CAVITIES IN THE HUMAN OR ANIMAL BODY
SONDE INTRACORPORELLE POUR L'ANALYSE, LE DIAGNOSTIC ET/OU LA THERAPIE, PAR EXEMPLE D'ORGANES CREUX OU DE CAVITES ORGANIQUES DANS LE CORPS HUMAIN OU ANIMAL

(30) Priorität: 14.09.2001 DE 10146197
(43) Veröffentlichungstag der Anmeldung: 16.06.2004
(73) Patentinhaber: Karl Storz GmbH & Co. KG, 78532 Tuttlingen (DE)
(72) Erfinder: IRION, Klaus, M., 78576 Liptingen (DE)
(74) Vertreter: Heuckeroth, Volker, Dr.
(86) Internationale Anmeldenummer: PCT/EP2002/010161
(87) Internationale Veröffentlichungsnummer: WO 2003/024328

(56) Entgegenhaltungen:
- WO-A-00/22975
- WO-A-02/36007
- US-A- 5 833 603
- US-B1- 6 240 312

## Beschreibung

Die Erfindung betrifft eine intrakorporale Sonde zur Untersuchung und/oder Therapie beispielsweise von Hohlorganen oder natürlichen oder künstlich geschaffenen Körperhöhlen im menschlichen oder tierischen Körper, wobei die Sonde in Form einer Kapsel ausgebildet ist, die in den Körper ohne externe Verbindungselemente einbringbar ist, und wobei die Sonde zumindest ein lichtabstrahlendes Element und zumindest ein lichtempfangendes Element aufweist, wobei das lichtempfangende Element Licht in einem anderen Wellenlängenbereich empfängt als das lichtabstrahlende Element abstrahlt.

Eine derartige intrakorporale Sonde ist aus US-A-5 833 603 bekannt.

Es ist bekannt, zur Untersuchung von Hohlorganen oder Körperhöhlen bildgebende Verfahren wie Ultraschall, Röntgen, Computertomographie und Magnetresonanz-Tomographie einzusetzen. Des weiteren sind für denselben Zweck endoskopische Verfahren bekannt.

Endoskope werden zur visuellen Darstellung des Körperinneren in den Körper eingebracht. Quantitative Informationen wie beispielsweise Informationen über den Sauerstoffgehalt, den Kohlendioxidgehalt oder den pH-Wert werden zum Teil über Sonden gewonnen, die über den Instrumentenkanal des Endoskops in das Körperinnere eingeführt werden, und die mit einem extrakorporalen Analysegerät verbunden sind.

Ein neueres Gebiet der medizinischen Analyse bzw. Diagnose betrifft die sogenannte photodynamische Diagnose. Bei der photodynamischen Diagnose werden photosensitive Substanzen, beispielsweise Aminolävulinsäure (ALA) oder deren Vorstufe in das zu untersuchende Gewebe bzw. in das zu untersuchende Gewebeareal eingebracht. Dabei wird der Effekt ausgenutzt, daß sich derartige photosensitive Substanzen in maligne Gewebe, beispielsweise Tumore, in größerem Ausmaß einlagern als in gesunde Gewebe. Mittels eines in den Körper eingeführten Fluoreszenzendoskops wird das zu untersuchende Gewebe mit Licht bestrahlt, wodurch die photosensitiven Substanzen zur Fluoreszenz angeregt werden. Das Auftreten von Fluoreszenz bzw. die Intensität der beobachteten Fluoreszenz ermöglicht dann eine Aussage darüber, ob das untersuchte Gewebe gesund oder pathologisch verändert ist. Mit dieser Methode lassen sich Tumore im Frühstadium visualisieren.

Die Nachteile derzeitiger endoskopischer Systeme bestehen darin, daß Endoskope nicht für die Untersuchung aller Körperbereiche eingesetzt werden können. So sind beispielsweise Bereiche des Dünndarms für ein Endoskop nicht zugänglich, nicht einmal für solche Endoskope, die einen flexiblen Schaft aufweisen. Des weiteren stellen endoskopische Untersuchungen keinen vernachlässigbaren Aufwand für den untersuchenden Arzt und auch für den Patienten dar, und sind daher für eine routinemäßige Untersuchung nicht einsetzbar. Die Einführung eines endoskopischen Schlauchs stellt für einen Patienten, beispielsweise bei gastroenterologischen Untersuchungen, eine erhebliche Belastung dar. Darüber hinaus ist eine quantitative Informationsgewinnung bei Standardendoskopen nicht gegeben und bedarf der Bild-Analyse durch den untersuchenden Arzt.

Dem gegenüber stellt die aus dem oben genannten Dokument US-A-5 833 603 bekannte intrakorporale Sonde eine den Patienten weniger beeinträchtigende Möglichkeit dar, Eigenschaften von Körperorganen zu untersuchen. Mit dieser bekannten intrakorporalen Sonde können optische, mechanische, chemische und elektrochemische Eigenschaften von Gewebearealen oder Körperorganen im menschlichen Körper untersucht werden. Diese bekannte Sonde kann mit Temperatursensoren, Drucksensoren, Magnetsensoren, Beschleunigungssensoren, Akustiksensoren, chemischen Sensoren usw. ausgestattet sein. Die Sonde wird von extrakorporal drahtlos mit Energie versorgt.

Des Weiteren ist aus dem Dokument US-A-6 240 312 eine intrakorporale Sonde bekannt, die fernsteuerbar ist, um Abnormalitäten innerhalb eines menschlichen Körpers zu diagnostizieren und/oder zu behandeln. Diese bekannte Sonde weist eine Mikro-Videokamera auf sowie eine Laserlichtquelle, um zweidimensionale Bildinformationen und spektroskopische Informationen, beispielsweise Fluoreszenz, Absorption, elastische Streuung, Ramanstreuung usw. innerhalb des Körpers zu gewinnen.

Aus dem Dokument WO 00/22975 ist eine intrakorporale Sonde bekannt, die eine Lichtquelle, ein Kamerasystem zur Aufnahme von Bildern und ein optisches System zum Fokussieren der Bilder auf das Kamerasystem aufweist. Ein Sender überträgt das Videosignal des Kamerasystems nach extrakorporal. Mittels des Kamerasystems wird von der Sonde intrakorporal eine "Landkarte" des Magen-Darm-Traktes aufgenommen. Beim zweiten Durchgang der Sonde durch den Magen-Darm-Trakt kann der Ort der Sonde durch eine Bildverarbeitung mittels eines Vergleiches des aktuellen von der Sonde gelieferten Bildes mit der zuvor aufgezeichneten "Landkarte" erfasst werden.

Aus der EP-A-0 667 115 ist eine intrakorporale Sonde bekannt, die in Form einer Kapsel ausgebildet ist, die vom zu untersuchenden Patienten geschluckt werden kann, um den Magen-Darm-Trakt visuell untersuchen zu können. Die von der Sonde empfangenen optischen Signale werden telemetrisch über einen in der Kapsel vorhandenen Sender nach extrakorporal übertragen und dort visualisiert. Diese bekannte autonome Videosonde ermöglicht es zwar, den Magen-Darm-Trakt visuell zu inspizieren und die Bilder telemetrisch nach außen zu übertragen, jedoch bleibt die Analyse des übertragenen Bildes dem erfahrenen Arzt vorbehalten. Der Arzt muß die Bilddaten der gesamten Passage der Videosonde durch den Magen-Darm-Trakt, die über acht Stunden dauern kann, auswerten bzw. beurteilen. Ein Großteil der Bilder besitzt aufgrund der willkürlichen Position im Hohlorgan keine auswertbare Information. Des weiteren ist an dieser bekannten Videosonde nachteilig, daß die Bildqualität durch die oberflächliche Verschmutzung der Optik durch Schleim usw. gemindert sein kann.

Der Erfindung liegt daher die Aufgabe zugrunde, eine intrakorporale Sonde der eingangs genannten Art dahingehend weiterzubilden, daß sie eine gezielte Erfassung diagnostisch relevanter optischer Informationen ohne aufwendige Bildanalyse ermöglicht.

Erfindungsgemäß wird diese Aufgabe hinsichtlich der eingangs genannten intrakorporalen Sonde dadurch gelöst, daß in der Kapsel ein Lageerfassungselement angeordnet ist, dessen Lage von extrakorporal bestimmt werden kann, um die Position der Sonde und die Lage der Sonde bezüglich ihrer eigenen Achsen relativ zu dem Hohlorgan oder der Körperhöhle zu erfassen.

Mit der erfindungsgemäßen intrakorporalen Sonde ist es möglich, diagnostisch relevante Informationen auf optischem Wege zu erfassen, ohne daß es jedoch einer optischen Abbildung bedarf. Dadurch, daß das zumindest eine lichtempfangende Element Licht in einem anderen Wellenlängenbereich empfängt als das zumindest eine lichtabstrahlende Element abstrahlt, ist es nämlich möglich, gewebespezifisch eingelagerte Photosensitizer wie bei der photodynamischen Diagnose mit dem Licht des lichtabstrahlenden Elements anzuregen, d.h. das lichtabstrahlende Element strahlt eine Anregungswellenlänge ab, und deren Fluoreszenz zu bestimmen, indem das zumindest eine lichtempfangende Element die Emissionswellenlänge der Fluoreszenz empfängt. Dies läßt eine Charakterisierung des Gewebezustands zu, ohne daß das Gewebe dazu visuell abgebildet werden muß. Im einfachsten Fall kann die durch die erfindungsgemäße intrakorporale Sonde erhaltene Information, die vorzugsweise telemetrisch nach außen übertragen wird, darin bestehen, ob eine Fluoreszenz detektiert (malignes Gewebe) oder nicht detektiert (gesundes Gewebe) wurde. Probleme einer unscharfen Bildübertragung, die sich durch eine verschmutzte Optik der intrakorporalen Sonde wie bei der bekannten intrakorporalen Sonde ergeben können, stellen sich dabei nicht. Der behandelnde Arzt muß kein visuelles Bild hinsichtlich eines pathologischen Zustands analysieren oder interpretieren, sondern allein das Vorhandensein eines Signals (Fluoreszenz vorhanden oder nicht vorhanden) liefert dem Arzt einen Befund. Als Photosensibilisatoren können körpereigene Stoffe (beispielsweise Flurophore) herangezogen werden, die eine Autofluoreszenz bewirken, oder es werden Photosensibilisatoren von außen verabreicht, die sich gewebespezifisch einlagern und Fluoreszenz emittieren. Zur Ermittlung des organzustandes können auch reine Fluoreszenzstoffe eingesetzt werden, die sich nicht gewebespezifisch einlagern, beispielsweise im Blut. Derartige Stoffe wie beispielsweise Natrium-Fluoreszein können zur Detektion von Blutungen, beispielsweise im Magen oder im Darm, eingesetzt werden, indem sie durch Injektion verabreicht werden. Mit der erfindungsgemäßen intrakorporalen Sonde wird eine insbesondere für die photodynamische Diagnose geeignete autonome Sonde bereitgestellt, die gegenüber den bekannten Endoskopiesystemen, die für den Patienten mitunter eine Belastung darstellen, eine höhere Akzeptanz erfährt.

In der Kapsel ist ein Lageerfassungselement angeordnet, dessen Lage von extrakorporal bestimmt werden kann.

Diese Maßnahme hat den Vorteil, daß die Lage oder Position der Sonde innerhalb des Körpers des Patienten verfolgt werden kann. Dies ist insbesondere dann von Vorteil, wenn die Sonde sich in einem Körperhohlraum des Patienten befindet, wo sie aufgrund der natürlichen Peristaltik nicht ortsfest im Körper liegt, sondern bewegt wird. Da bei einer derartigen Bewegung auch die räumliche Lage der Sonde um ihre sondeneigenen Achsen verändert wird, kann über die Lageerfassung auch die Lage der Sonde bezüglich ihrer eigenen Achsen und somit auch die Lage relativ zu dem zu untersuchenden Gewebe stets verfolgt werden.

Die beschriebene Sonde kann auch als Implantat im Körper verbleiben. Dies ermöglicht beispielsweise eine intrakorporale postoperative Therapiekontrolle.

In einer bevorzugten Ausgestaltung ist das Licht des zumindest einen lichtabstrahlenden Elements kurzwelliger als das Licht, das durch das zumindest eine lichtempfangende Element empfangbar ist.

In dieser Ausgestaltung eignet sich die erfindungsgemäße intrakorporale Sonde vorteilhafterweise insbesondere für einen Einsatz im Rahmen der photodynamischen Diagnose, da mit dem kurzwelligeren abgestrahlten Licht körpereigene oder zugeführte photosensitive Substanzen zur Fluoreszenz angeregt werden können, und das Fluoreszenzlicht dann durch das lichtempfangende Element empfangen werden kann, wobei das empfangene Licht vom abgestrahlten Licht spektral wohl getrennt ist, so daß keine Fehlinterpretationen der von der Sonde gelieferten Daten möglich sind.

In einer weiteren bevorzugten Ausgestaltung weist das zumindest eine lichtabstrahlende Element eine den gesamten Raumwinkel erfassende Abstrahlcharakteristik auf, und/oder ist in der Kapsel eine Mehrzahl von lichtabstrahlenden Elementen derart angeordnet, daß die Lichtabstrahlung den gesamten Raumwinkel erfaßt.

Die vorstehend genannten Maßnahmen, die alternativ oder in Verbindung miteinander bei der erfindungsgemäßen intrakorporalen Sonde vorgesehen sein können, haben den Vorteil, daß die Abstrahlung des oder der lichtabstrahlenden Elemente weitestgehend unabhängig von der Lage der Sonde im Hohlorgan oder in der Körperhöhle gleichmäßig in alle Raumrichtungen erfolgt.

In einer weiteren bevorzugten Ausgestaltung ist das zumindest eine lichtabstrahlende Element eine Leuchtdiode (LED).

Hierbei ist von Vorteil, daß durch die Verwendung von Leuchtdioden als lichtabstrahlende Elemente die intrakorporale Sonde mit geringen Kosten herstellbar ist. Des weiteren haben Leuchtdioden den Vorteil einer hohen Lichtausbeute.

In einer weiteren bevorzugten Ausgestaltung strahlt die Leuchtdiode im blauen Frequenzbereich ab.

Eine im blauen Frequenzbereich abstrahlende Leuchtdiode eignet sich besonders gut zur Anregung von Fluoreszenz und damit zur Verwendung der erfindungsgemäßen intrakorporalen Sonde zur Fluoreszenzdiagnose von Gewebe im menschlichen oder tierischen Körper.

In einer weiteren bevorzugten Ausgestaltung ist das zumindest eine lichtempfangende Element derart ausgebildet, daß es Licht aus dem gesamten Raumwinkelbereich empfängt, und/oder ist in der Kapsel eine Mehrzahl von lichtempfangenden Elementen derart angeordnet, daß Licht aus dem gesamten Raumwinkelbereich empfangbar ist.

Bei diesen zuvor genannten Maßnahmen, die alternativ oder in Verbindung miteinander bei der erfindungsgemäßen Sonde vorgesehen sein können, besteht wiederum der Vorteil darin, daß die Sonde das von dem zu untersuchenden Gewebe kommende Licht weitestgehend unabhängig von der Lage der Sonde im Körper gleichmäßig empfangen kann.

In einer weiteren bevorzugten Ausgestaltung ist an dem zumindest einen lichtabstrahlenden Element und/oder an dem zumindest einen lichtempfangenden Element ein optisches Filterelement angeordnet.

Diese Maßnahme hat den Vorteil, daß eine besonders gute Trennung der Wellenlängenbereiche zwischen dem abgestrahlten und dem empfangenen Licht erreicht wird, so daß insbesondere das zumindest eine lichtempfangende Element nur das für die Diagnose relevante Licht empfängt. Als optische Filter können insbesondere Interferenzfilter vor den lichtabstrahlenden und lichtempfangenden Elementen aufgebracht sein.

In einer weiteren bevorzugten Ausgestaltung ist in der Kapsel zumindest ein lichtempfangendes Element in Form eines Bildsensors, insbesondere eines zweidimensionalen Bildsensors, zum Empfangen eines visuellen Bildes vorhanden.

Hierbei ist von Vorteil, daß neben der durch das zumindest eine lichtempfangende Element gewonnenen spektralen Information zusätzlich eine bildgebende Information erhalten werden kann, die eine Visualisierung des untersuchten Hohlorgans oder der untersuchten Körperhöhle ermöglicht. Wie jedoch zuvor erläutert, liegt das Hauptaugenmerk der vorliegenden Erfindung nicht auf der visuellen Darstellung, sondern auf der Gewinnung optischer Informationen, die keine visuelle Darstellung des untersuchten Gewebeareals erfordern. Die visuelle Darstellung des untersuchten Gewebeareals hat jedoch den zusätzlichen Vorteil einer leichteren Orientierung für den Arzt.

Entsprechend ist es in einer weiteren bevorzugten Ausgestaltung vorgesehen, daß in der Kapsel zumindest ein lichtabstrahlendes Element angeordnet ist, das Weißlicht abstrahlt.

Hierbei ist von Vorteil, daß eine Ausleuchtung des Körperhohlraums bzw. des Hohlorgans mit Weißlicht eine naturgetreuere Visualisierung des untersuchten Gewebeareals ermöglicht, als dies durch Ausleuchtung mit farbigem Licht der Fall ist.

In einer weiteren bevorzugten Ausgestaltung ist in der Kapsel ein Senderelement zum Abstrahlen von Signalen von der Sonde nach extrakorporal angeordnet.

Hierbei ist von Vorteil, daß die von der Sonde empfangene und ggf. durch eine Signalverarbeitungseinheit innerhalb der Kapsel aufbereitete optische Information instantan über eine entsprechende Empfangseinheit und eine damit verbundene weitere Darstellungseinheit dem behandelnden Arzt zur Verfügung stehen, währenddessen die Sonde im Körper verbleiben kann.

Dabei ist weiter bevorzugt, wenn in der Kapsel ein Signalvorverarbeitungselement angeordnet ist, daß das von dem zumindest einen lichtempfangenden Element opto-elektrische Signal an das Senderelement weitergibt.

Ein derartiges Signalvorverarbeitungselement hat den Vorteil, eine Vorverarbeitung der von dem zumindest einen lichtempfangenden Element empfangenen Lichtsignale zu ermöglichen, so daß dem Arzt nur die für die Diagnose relevanten Signale übermittelt werden. Des weiteren kann das Signalvorverarbeitungselement bei einem Pulsbetrieb des zumindest einen lichtabstrahlenden Elements die Synchronisation zwischen der gepulsten Abstrahlung und dem Empfang des Lichtes durch das zumindest eine lichtempfangende Element vornehmen.

In einer weiteren bevorzugten Ausgestaltung ist in der Kapsel ein Signalspeicher zum Abspeichern von Signalen des zumindest einen lichtempfangenden Elements angeordnet.

Diese Maßnahme ist insbesondere dann von Vorteil, wenn die von dem zumindest einen lichtempfangenden Element empfangenen Lichtsignale nicht sofort verarbeitet oder nicht sofort nach extrakorporal telemetrisch übermittelt werden sollen, sondern wenn eine Auswertung der von der Sonde aufgenommenen Daten zu einem späteren Zeitpunkt vorgenommen werden soll. Dies hat insbesondere den Vorteil, daß zeitgleich mehrere Patienten mit einer erfindungsgemäßen intrakorporalen Sonde diagnostiziert werden können, und die einzelnen intrakorporalen Sonden bzw. deren Daten nach Entnahme der Sonde aus dem Körper dann ausgelesen und ausgewertet werden.

Es ist weiter bevorzugt, wenn das Lageerfassungselement als Spulensystem ausgebildet ist, dessen Position über einen externen Magnetfelddetektor erfaßbar ist.

Ein derartiges Spulensystem als Lageerfassungselement läßt sich vorteilhafterweise miniaturisiert ausgestalten, so daß es sich besonders vorteilhaft zur Ausbildung einer miniaturisierten intrakorporalen Sonde eignet.

Die Lokalisierung der Sonde im Körper ist vor allem dann notwendig, wenn die Sonde ein positives Analyse- bzw. Diagnoseergebnis zeigt. In diesem Fall kann es vorgesehen sein, daß magnetische Spulensystem elektrisch zu aktivieren, um die Position der Sonde im Körper zu ermitteln. Selbstverständlich setzt ein derartiges Spulensystem voraus, daß die Außenwand der Kapsel bzw. die Hülle der Kapsel nicht metallisch ausgeführt ist, und daß auch innerhalb der Kapsel möglichst wenig Metall zum Einsatz kommt.

In einer weiteren bevorzugten Ausgestaltung ist in der Kapsel ein Positionierungselement angeordnet, das von extrakorporal zur Positionierung der Sonde ansteuerbar ist.

Diese Maßnahme hat den Vorteil, daß die Sonde an einer gewünschten Stelle und in einer bestimmten Lage innerhalb des Körperhohlraumes bzw. Hohlorgans lagefest positioniert werden kann. Dies ist insbesondere in Körperhohlräumen bzw. Körperorganen von Vorteil, die einen im Vergleich zur Abmessung der Sonde wesentlich größeren Querschnitt aufweisen, so daß es ohne eine derartige Positionierungsmaßnahme nicht möglich wäre, die Sonde an einer vorbestimmten Position und in einer bestimmten Lage zu fixieren. Insbesondere in Körperorganen, die eine peristaltik besitzen, hat diese Maßnahme den Vorteil einer gezielteren und exakteren Diagnose. In einer weiteren bevorzugten Ausgestaltung ist in der Kapsel ein Energieversorgungselement oder ein Element zum Empfangen von extrakorporal eingestrahlter elektromagnetischer Energie angeordnet.

Hierbei ist von Vorteil, daß die erfindungsgemäße intrakorporale Sonde hinsichtlich ihrer Energieversorgung autonom ist, d.h. keine externen Verbindungsleitungen zur Energieversorgung benötigt. Das Energieversorgungselement kann beispielsweise eine miniaturisierte Batterie oder ein Energiespeicher mit Energiewandler sein, der in elektrische Energie transformiert.

In einer weiteren bevorzugten Ausgestaltung sind auf der Kapsel fluoriszierende/leuchtende Markerstoffe aufgebracht.

Diese Maßnahme hat den Vorteil, daß die Markerstoffe in Wechselwirkung mit der Umgebung, beispielsweise Blut, treten können, so daß Parameter wie Sauerstoffgehalt, Kohlendioxidgehalt, usw. abgeleitet werden können.

Die Kapsel kann auch bevorzugt einen Leuchtstoff aufweisen, der extrakorporal bspw. über elektromagnetische Energie zum Leuchten angeregt wird und über die transparente Kapselhülle abstrahlt. Diese Anregung kann extrakorporal auch über Röntgenstrahlung erfolgen, indem ein röntgensensitiver Farbstoff verwendet wird, oder die Anregung kann extrakorporal über Ultraschallenergie erfolgen, indem photoakustische Farbstoffe verwendet werden. Allgemein können als Farbstoffe beispielsweise Natrium-Fluoreszein (Phatalein), Eosin, Rodamin sowie deren Derivate verwendet werden. Ein solcher Leuchtstoff innerhalb der Kapsel dient dann als das zumindest eine lichtabstrahlende Element, das keine in der Kapsel vorhandene Energieversorgung benötigt.

In einer weiteren bevorzugten Ausgestaltung ist in der Kapsel ein Reservoir für therapeutische Substanzen und/oder diagnostische Substanzen vorhanden, die intrakorporal von der Sonde abgegeben werden.

Hierbei ist von Vorteil, daß zusammen mit der Sonde die zuvor genannten Substanzen in den Körper eingebracht werden können, so daß für das Einbringen dieser Substanzen kein zusätzlicher Behandlungsschritt erforderlich ist, und außerdem die Substanzen gezielter an den Ort gebracht werden können, wo auch die Untersuchung mittels der Sonde erfolgt.

Zur Therapie von malignen oder prämalignen Veränderungen kann ein photodynamisch-therapeutisch wirkender Photosensibilisator beigegeben werden, der unter Lichtbestrahlung therapeutisch wirksam wird. Im übrigen können als photosensitive Substanzen, die für die Diagnose und/oder die Therapie geeignet sind, beispielsweise Porphyrine (Protoporphyrin IX, beispielsweise induziert durch Aminolävulinsäure (ALA), Benzoporphyrin), Metatetrahydrophenylchlorine (m-THPC), Cyanine (Phtalocyanine (Znphtalocyanin)), Hypericin, Tinethyletiopurpurine (SnET₂), Luthetiumtexaphyrine sowie deren Derivate oder andere zum Einsatz kommen.

In einer weiteren bevorzugten Ausgestaltung ist in der Kapsel ein Ultraschallsender-/-empfängerelement zur Ultraschallbildgebung angeordnet.

Hierbei ist von Vorteil, daß über die Ultraschallbildgebung eine Schnittbilderfassung tieferliegender Gewebebereiche ermöglicht wird, die wegen der geringeren Eindringtiefe von Licht in Gewebe mit optischen Mitteln nicht oder nur unzureichend erfaßt werden können.

In einer weiteren bevorzugten Ausgestaltung weist die Sonde zumindest eine Leitung nach extrakorporal zum Austausch von Informationen, Energie und/oder Substanzen auf.

In einer weiteren bevorzugten Ausgestaltung ist die Sonde als Implantat ausgeführt, und eine Kapselwand ist mit Langzeit-biokompatiblem und sterilisierbarem Material ausgeführt.

Hierbei ist von Vorteil, daß die Sonde auch als Langzeitimplantat ausgeführt werden kann, beispielsweise zur Implantation in ein zuvor reseziertes Tumorbett, indem die Kapselwand biokompatibel und sterilisierbar ausgeführt ist. Für eine Verwendung der Sonde als Langzeitimplantat sollte die Sonde darüber hinaus stabil ausgeführt sein. Eine beispielhafte Applikation für ein derartiges Sondenimplantat besteht beispielsweise beim (schwergradigen) Glioblastom in der Neurochirurgie. Bei diesem kritischen Krankheitsbild ist es meist nicht möglich, durch eine Operation alle bestehenden Tumorbestandteile bzw. tiefer liegenden Tumorzellen zu resezieren bzw. herauszunehmen. Mit einer Ausführung der erfindungsgemäßen Sonde als Langzeitimplantat kann auch nach der Operation eine Therapiefortsetzung intrakorporal ermöglicht werden.

In einer weiteren bevorzugten Ausgestaltung weist die Sonde als Implantat eine voll umschließende transparente Kapselwand auf, die vorzugsweise aus Glas besteht.

Wenn die gesamte Kapselwand als stabile Glaswandung ausgeführt ist, hat dies den Vorteil, daß eine Transparenz und Biokompatibilität per se gegeben sind. Durch eine ggf. vorhandene Integration von lichtstreuenden Körpern kann die Homogenität der Abstrahlung durch die Kapselwand weiter verbessert werden. Glas als Material für die Kapselwand bietet darüber hinaus vorteilhafterweise einen gewissen Schutz der integrierten Bauteile gegenüber energiereicher ("harter") Strahlung, die zur postoperativen Weiterbehandlung oft eingesetzt wird, so daß sich die Sonde auch bezüglich dieses Aspektes als Langzeitimplantat eignet.

Wie bereits erwähnt, ist die erfindungsgemäße intrakorporale Sonde nicht nur für diagnostische Zwecke geeignet, sondern auch zusätzlich für therapeutische Zwecke, wie in einer der zuvor genannten Ausgestaltungen beschrieben wurde.

Dazu ist es in einer weiteren bevorzugten Ausgestaltung vorgesehen, daß in der Kapsel zumindest ein Therapielicht abstrahlendes Element oder Elementarray angeordnet ist, das Licht zur photodynamischen Therapie hauptsächlich in dem Wellenlängenbereich abstrahlt, in dem das Absorptionsmaximum eines in den Körper eingebrachten Photosensitizers liegt.

Diese Maßnahme hat den Vorteil, daß durch die Integration von Therapielicht abstrahlenden Elementen, insbesondere von solchen, deren Wellenlänge im Absorptionsmaximum des Photosensitizers liegt, eine optimale Wirkung der photodynamischen Therapie erzielt werden kann.

In einer weiteren bevorzugten Ausgestaltung ist das zumindest eine Therapielicht abstrahlende Element bzw. Elementarray in der Kapsel so angeordnet, daß der gesamte Raumwinkel homogen ausgeleuchtet wird.

Hierbei ist von Vorteil, daß der gesamte Raumwinkel des zu behandelnden Körperareals homogen mit Therapielicht bestrahlt werden kann.

In einer weiteren bevorzugten Ausgestaltung sind das zumindest eine lichtabstrahlendes Element, das zumindest eine lichtempfangendes Element und das zumindest eine Therapielicht abstrahlendes Element so ausgerichtet, daß lokal in dem Raumwinkel Therapielicht abgestrahlt werden kann, in dem ein Fluoreszenzsignal von dem zumindest einen lichtempfangenden Element empfangen wird, das von dem durch das zumindest ein lichtabstrahlendes Element abgestrahlten Anregungslicht hervorgerufen ist.

Diese Maßnahme hat den Vorteil, daß nur zuvor aufgrund der Diagnose mittels der lichtabstrahlenden und lichtempfangenden Elemente als maligne Bereiche, d.h. fluoreszierende Bereiche, detektierte Gewebeareale anschließend mit Therapielicht im Raumwinkel bestrahlt werden, d.h. mit dieser Anordnung wird eine lokal gerichtete Therapie ermöglicht.

In einer weiteren bevorzugten Ausgestaltung ist das zumindest eine Therapielicht abstrahlende Element als Leuchtdiode mit einer Wellenlänge im Bereich von 590 bis 650 nm ausgebildet.

Diese Maßnahme hat den Vorteil, daß in diesem Wellenlängenbereich die Absorptionsmaxima vieler Photosensitizer wie beispielsweise Aminolävolinsäure (ALA), Hypericin usw. liegen, und daß die Leistung der Leuchtdioden im Vergleich zu kurzwelliger abstrahlenden Leuchtdioden relativ hoch ist.

Weitere Vorteile oder Merkmale ergeben sich aus der nachfolgenden Beschreibung und der beigefügten Zeichnung. Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese hiernach näher beschrieben. Es zeigen:
- Fig. 1: eine schematische Darstellung einer intrakorporalen Sonde in Seitenansicht in stark vergrößertem Maßstab;
- Fig. 2: einen Schnitt durch die intrakorporale Sonde in Fig. 1 entlang der Linie II-II in Fig. 1; und
- Fig. 3: eine schematische Darstellung, die die intrakorporale Sonde in Fig. 1 in einem Hohlorgan eines menschlichen Körpers zeigt.

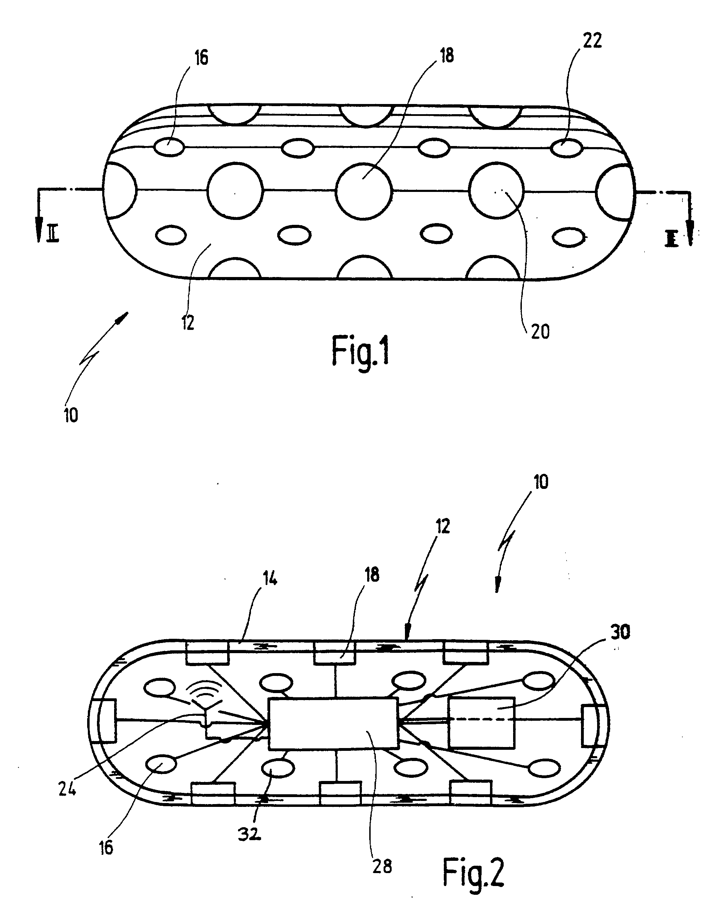

In Figuren 1 und 2 ist eine mit dem allgemeinen Bezugszeichen 10 versehene intrakorporale Sonde zur Untersuchung beispielsweise von Hohlorganen oder Körperhöhlen im menschlichen oder tierischen Körper dargestellt. Die Sonde 10 dient insbesondere als autonome Sonde zur Verwendung in der photodynamischen Diagnose. Die Kapsel 12 weist eine vorzugsweise transparente Kapselhülle bzw. Kapselwand 14 auf.

In der Kapsel ist eine Mehrzahl von lichtabstrahlenden Elementen 16 angeordnet, die als Leuchtdioden ausgebildet sind. Die lichtabstrahlenden Elemente 16 sind derart verteilt in der Kapsel angeordnet, daß die Lichtabstrahlung den gesamten Raumwinkel erfaßt, d.h. daß die Sonde 10 Licht in allen Raumrichtungen, und zwar möglichst gleichmäßig, abstrahlt.

Die als Leuchtdioden ausgebildeten lichtabstrahlenden Elemente 16 strahlen Licht im blauen Frequenzbereich ab, beispielsweise bei einer Wellenlänge von etwa 480 nm, bei der Verwendung von Natrium-Fluoreszein.

Des weiteren sind in der Kapsel 12 lichtempfangende Elemente 18 in Form von photoelektrischen Elementen zum Empfangen von Licht angeordnet. Die lichtempfangenden Elemente 18 sind in der Lage, Licht in einem anderen Wellenlängenbereich zu empfangen, als die lichtabstrahlenden Elemente abstrahlen. Die spektralen Eigenschaften der lichtempfangenden Elemente 18 sind demnach von den spektralen Eigenschaften der lichtabstrahlenden Elemente 16 getrennt. Im vorliegenden Ausführungsbeispiel beginnt die Empfindlichkeit der lichtempfangenen Elemente 18 bei einer Wellenlänge von > 500 nm, bei der Verwendung von Natrium-Fluoreszein.

Die Lichtempfindlichkeit der lichtempfangenden Elemente 18 ist in einem Wellenlängenbereich gegeben, der langwelliger ist als das Licht, das durch die lichtabstrahlenden Elemente 16 abgestrahlt wird.

Zur noch verbesserten Trennung der Spektralbereiche können vor den lichtabstrahlenden Elementen 16 und/oder vor den lichtempfangenden Elementen 18 optische Filterelemente angeordnet sein, beispielsweise in Form von Interferenzfiltern.

Die lichtempfangenden Elemente 18 sind derart in der Kapsel verteilt angeordnet, daß sie Licht aus dem gesamten Raumwinkelbereich empfangen können.

Eines oder mehrere der lichtempfangenden Elemente 18 kann auch in Form eines Bildsensors, insbesondere eines zweidimensionalen Bildsensors, zum Empfangen eines visuellen Bildes ausgebildet sein, wie beispielhaft für ein lichtempfangendes Element 20 in Fig. 1 dargestellt ist.

Das lichtempfangende Element 20 kann beispielsweise ein CCD-Sensor oder ein CMOS-Sensor sein.

Entsprechend kann auch eines der lichtabstrahlenden Elemente 16, wie beispielsweise für ein lichtabstrahlendes Element 22 beispielhaft dargestellt ist, als Weißlichtquelle ausgebildet sein, um eine möglichst naturgetreue Ausleuchtung des Beobachtungsareals im menschlichen oder tierischen Körper zu bewirken. Das Element 22 ist vorzugsweise eine weiß leuchtende Leuchtdiode.

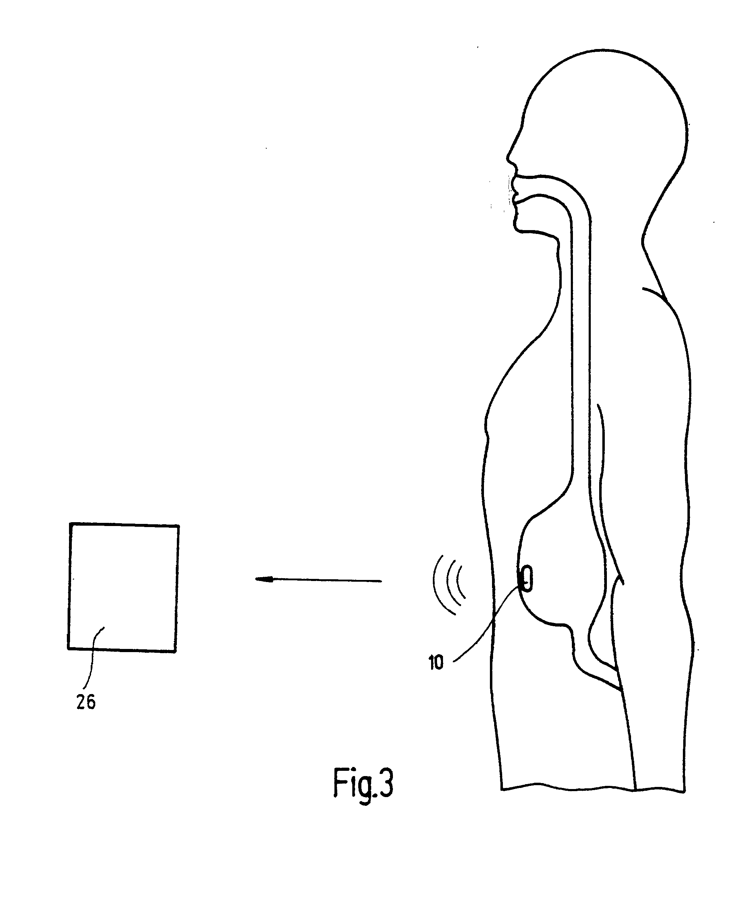

Des weiteren ist in der Kapsel 12 ein Senderelement 24 angeordnet, das Signale von der Sonde nach extrakorporal abstrahlt, wie in Fig. 3 schematisch dargestellt ist. Die von der Sonde 10 abgestrahlten Signale werden dann von einem extrakorporalen Empfänger 26 empfangen und dem behandelnden Arzt beispielsweise auf einer Display-Einheit oder einem Bildschirm zur Darstellung gebracht.

Ein Signalvorverarbeitungselement 28 ist gemäß Fig. 2 ebenfalls in der Kapsel 12 angeordnet. Das Signalvorverarbeitungselement 28 gibt die von den lichtempfangenden Elementen 18 kommenden opto-elektrischen Signale an das Senderelement 24 weiter. Entsprechend sind alle lichtempfangenden Elemente 18 elektrisch mit dem Signalvorverarbeitungselement 28 verbunden. Auch die lichtabstrahlenden Elemente 16 sind alle mit dem Signalvorverarbeitungselement 28 verbunden. So kann beispielsweise das Signalvorverarbeitungselement beim Pulsbetrieb der lichtabstrahlenden Elemente 16 auch die Synchronisation zwischen der gepulsten Lichtabstrahlung und der Erfassung der Signale der lichtempfangenden Elemente 18 vornehmen.

Alternativ oder zusätzlich zu dem Senderelement 24 kann in der Kapsel ein nicht dargestellter Signalspeicher zum Abspeichern von Signalen der lichtempfangenden Elemente 18 angeordnet sein.

Des weiteren ist in der Kapsel 12 ein Energieversorgungselement 30 angeordnet, das der Energieversorgung der lichtabstrahlenden Elemente 16, der lichtempfangenden Elemente 18 dient, und das mit dem Signalvorverarbeitungselement 28 verbunden ist. Das Energieversorgungselement 30 ist beispielsweise eine Mikrobatterie, kann jedoch auch ein Element zum Empfangen von extrakorporal eingestrahlter elektromagnetischer Energie sein.

Um die Lage bzw. Position der Sonde 10 im menschlichen Körper nach deren Einbringen verfolgen zu können, ist in der Kapsel ein nicht dargestelltes Lageerfassungselement angeordnet, dessen Lage von extrakorporal bestimmt werden kann. Ein derartiges Lageerfassungselement ist vorzugsweise als Spulensystem ausgebildet, dessen Position über einen externen Magnetfelddetektor erfaßbar ist. Es versteht sich, daß die Kapselwand 14 entsprechendso ausgebildet ist, daß sie nicht magnetisch abschirmend wirkt.

In Fig. 3 ist die Sonde 10 in einer Position und einer Lage im Magen eines Patienten dargestellt. Um die Sonde 10 in der gezeigten Position und Lage zu fixieren, ist des weiteren vorzugsweise in der Kapsel ein Positionierungselement angeordnet, das von extrakorporal zur Positionierung der Sonde ansteuerbar ist. Eine derartige Positionierung kann beispielsweise von extrakorporal über die Wirkung eines Magnetfeldes erfolgen.

Des weiteren kann in der Kapsel ein Reservoir für therapeutische Substanzen und/oder diagnostische Substanzen vorhanden sein, wobei diese Substanzen dann intrakorporal von der Sonde allmählich abgegeben werden, beispielsweise durch eine nicht dargestellte Membran in der Kapselwand 14.

Schließlich kann in der Sonde 10 ein Ultraschallsender/Empfängerelement zur Ultraschallbildgebung angeordnet sein.

Auf der Außenseite der Kapsel 12 sind vorzugsweise fluoreszierende bzw. leuchtende Markerstoffe aufgebracht, wobei als derartiger Farbstoff Natrium-Fluoreszein verwendet werden kann.

Mit einem derartigen Farbstoff können Blutungen detektiert werden.

In der Kapsel 12 können auch Leuchtstoffe vorhanden sein, die durch Anregung von extrakorporal, beispielsweise über elektromagnetische Energie, über Röntgenstrahlung oder über Ultraschallenergie zum Leuchten angeregt werden und damit zur Lichtabstrahlung dienen können.

Bei einer Anwendung der intrakorporalen Sonde 10 für die photodynamische Diagnose wird mittels der lichtabstrahlenden Elemente 16 ein Photosensibilisator, der ein körpereigener Stoff oder ein von außen verabreichter Stoff sein kann, der sich gewebespezifisch einlagert, zur Fluoreszenz angeregt und das Fluoreszenzlicht wird dann mittels der lichtempfangenden Elemente 18 empfangen. Für die Beurteilung, ob das untersuchte Gewebe gesund oder pathologisch verändert ist, reicht es aus, wenn von der Sonde 10 nach extrakorporal ein Signal gesendet wird, das die Information 'Fluoreszenz vorhanden' (pathologisch verändertes Gewebe) oder 'Fluoreszenz nicht vorhanden' (gesundes Gewebe) enthält.

Dem Arzt wird somit ein Befund ermöglicht, ohne das das untersuchte Gewebe visuell abgebildet wird.

Des weiteren kann die Sonde 10 als Implantat ausgeführt sein, wobei dann die Kapselwand 14 mit Langzeit-biokompatiblem und sterilisierbarem Material ausgeführt ist. Die Kapselwand 14 der Sonde 10 ist insbesondere transparent ausgeführt und besteht vorzugsweise aus Glas, wobei Streupartikel in das Glas integriert sein können.

In der Kapsel 12 kann weiterhin zumindest ein Therapielicht abstrahlendes Element 32 angeordnet sein, das Licht zur photodynamischen Therapie abstrahlt, und zwar hauptsächlich in dem Wellenlängenbereich, in dem das Absorptionsmaximum eines Photosensitizers liegt, der zuvor in den Körper des Patienten eingebracht wurde.

Auch bezüglich des zumindest einen Therapielicht abstrahlenden Elements 32, das auch als Elementarray ausgebildet sein kann, ist es vorgesehen, daß der gesamte Raumwinkel homogen mit Therapielicht ausgeleuchtet wird. Dabei ist es vorteilhaft, wenn das zumindest eine Therapielicht abstrahlende Element 32, das zumindest eine lichtabstrahlende Element 16 und das zumindest eine lichtempfangende Element 18 so ausgerichtet sind, daß das Therapielicht von dem zumindest einen Therapielicht abstrahlenden Element 32 in einen Raumwinkel abgestrahlt wird, in dem zuvor ein Fluoreszenzsignal von dem zumindest einen lichtempfangenden Element 18 empfangen wurde, das Folge einer Anregung des von dem zumindest einen lichtabstrahlenden Element 16 emittierten Lichtes ist.

Das zumindest eine Therapielicht abstrahlende Element ist vorzugsweise eine Leuchtdiode mit einer Emissionswellenlänge im Bereich von 590 bis 650 nm.

Anwendungsfälle für die intrakorporale Sonde 10 können die Untersuchung des gastrointestinalen Trakts auf maligne Veränderungen unter Gabe eines tumorspezifisch sich anreichernden Photosensibilisators, die Registrierung von Blutungen im Magen, Colon und vor allem im Dünndarm sowie nach Operationen unter intravenöser Gabe eines Fluoreszenzfarbstoffes, die photodynamische Therapie von malignen oder prämalignen Gewebe durch Lichtexposition unter Gabe eines photodynamisch/therapeutisch wirkenden Photosensibilisators, oder die Therapienachkontrolle (beispielsweise Erkennung von Blutungen) nach offenen und minimal-invasiven Eingriffen sein, um einige Beispiele zu nennen.

## Patentansprüche

1. Intrakorporale Sonde zur Untersuchung und/oder Therapie beispielsweise von Hohlorganen oder natürlichen oder künstlich geschaffenen Körperhöhlen im menschlichen oder tierischen Körper, wobei die Sonde (10) in Form einer Kapsel (12) ausgebildet ist, die in den Körper ohne externe Verbindungselemente einbringbar ist, und wobei die Sonde (10) zumindest ein lichtabstrahlendes Element (16) und zumindest ein lichtempfangendes Element (18) aufweist, wobei das lichtempfangende Element (18) Licht in einem anderen Wellenlängenbereich empfängt als das lichtabstrahlende (16) Element abstrahlt, **dadurch gekennzeichnet, daß** in der Kapsel (12) ein Lageerfassungselement angeordnet ist, dessen Lage von extrakorporal bestimmt werden kann, um die Position der Sonde (10) und die Lage der Sonde (10) bezüglich ihrer eigenen Achsen relativ zu dem Hohlorgan oder der Körperhöhle zu erfassen.

2. Sonde nach Anspruch 1, **dadurch gekennzeichnet, daß** das Licht des zumindest einen lichtabstrahlenden Elements (16) kurzwelliger ist als das Licht, das durch das zumindest eine lichtempfangende Element (18) empfangbar ist.

3. Sonde nach Anspruch 1 oder 2, **dadurch gekennzeichnet, daß** das zumindest eine lichtabstrahlende Element (16) eine den gesamten Raumwinkel erfassende Abstrahlcharakteristik aufweist.

4. Sonde nach einem der Ansprüche 1 bis 3, **dadurch gekennzeichnet, daß** in der Kapsel (12) eine Mehrzahl von lichtabstrahlenden Elementen (16) derart angeordnet ist, daß die Lichtabstrahlung den gesamten Raumwinkel erfaßt.

5. Sonde nach einem der Ansprüche 1 bis 4, **dadurch gekennzeichnet, daß** das zumindest eine lichtabstrahlende Element (16) eine Leuchtdiode ist.

6. Sonde nach Anspruch 5, **dadurch gekennzeichnet, daß** die Leuchtdiode im blauen Frequenzbereich abstrahlt.

7. Sonde nach einem der Ansprüche 1 bis 6, **dadurch gekennzeichnet, daß** das zumindest eine lichtempfangende Element (18) derart ausgebildet ist, das es Licht aus dem gesamten Raumwinkelbereich empfängt.

8. Sonde nach einem der Ansprüche 1 bis 7, **dadurch gekennzeichnet, daß** in der Kapsel (12) eine Mehrzahl von lichtempfangenden Elementen (18) derart angeordnet ist, daß Licht aus dem gesamten Raumwinkelbereich empfangbar ist.

9. Sonde nach einem der Ansprüche 1 bis 8, **dadurch gekennzeichnet, daß** an dem zumindest einen lichtabstrahlenden Element (16) und/oder an dem zumindest einen lichtempfangenden Element (18) ein optisches Filterelement angeordnet ist.

10. Sonde nach einem der Ansprüche 1 bis 9, **dadurch gekennzeichnet, daß** in der Kapsel (12) zumindest ein lichtempfangendes Element (20) in Form eines Bildsensors, insbesondere eines zweidimensionalen Bildsensors, zum Empfangen eines visuellen Bildes vorhanden ist.

11. Sonde nach Anspruch 10, **dadurch gekennzeichnet, daß** in der Kapsel (12) zumindest ein lichtabstrahlendes Element (22) angeordnet ist, das Weißlicht abstrahlt.

12. Sonde nach einem der Ansprüche 1 bis 11, **dadurch gekennzeichnet, daß** in der Kapsel (12) ein Senderelement (26) zum Abstrahlen von Signalen von der Sonde (10) nach extrakorporal angeordnet ist.

13. Sonde nach Anspruch 12, **dadurch gekennzeichnet, daß** in der Kapsel ein Signalvorverarbeitungselement (28) vorhanden ist, daß das von dem zumindest einen lichtempfangenden Element (18) stammende opto-elektrische Signal an das Senderelement weitergibt.

14. Sonde nach einem der Ansprüche 1 bis 13, **dadurch gekennzeichnet, daß** in der Kapsel (12) ein Signalspeicher zum Abspeichern von Signalen des zumindest einen lichtempfangenden Elements (18) angeordnet ist.

15. Sonde nach einem der Ansprüche 1 bis 14, **dadurch gekennzeichnet, daß** das Lageerfassungselement als Spulensystem ausgebildet ist, dessen Position über einen externen Magnetfelddetektor erfaßbar ist.

16. Sonde nach einem der Ansprüche 1 bis 15, **dadurch gekennzeichnet, daß** in der Kapsel (12) ein Positionierungselement angeordnet ist, das von extrakorporal zur Positionierung der Sonde ansteuerbar ist.

17. Sonde nach einem der Ansprüche 1 bis 16, **dadurch gekennzeichnet, daß** in der Kapsel ein Energieversorgungselement (30) oder ein Element zum Empfangen von extrakorporal eingestrahlter elektromagnetischer Energie angeordnet ist.

18. Sonde nach einem der Ansprüche 1 bis 17, **dadurch gekennzeichnet, daß** auf der Kapsel (12) fluoreszierende/leuchtende Markerstoffe aufgebracht sind.

19. Sonde nach einem der Ansprüche 1 bis 18, **dadurch gekennzeichnet, daß** in der Kapsel (12) zumindest ein Leuchtstoff vorhanden ist, der durch Anregung extrakorporal anregbar ist und durch eine Kapselwand (14) abstrahlt.

20. Sonde nach einem der Ansprüche 1 bis 19, **dadurch gekennzeichnet, daß** in der Kapsel ein Reservoir für therapeutisiche Substanzen und/oder diagnostische Substanzen vorhanden ist, die intrakorporal von der Sonde abgegeben werden.

21. Sonde nach einem der Ansprüche 1 bis 20, **dadurch gekennzeichnet, daß** in der Sonde ein Ultraschallsender-/-empfängerelement zur Ultraschallbildgebung angeordnet ist.

22. Sonde nach einem der Ansprüche 1 bis 21, **dadurch gekennzeichnet, daß** sie zumindest eine Leitung nach extrakorporal zum Austausch von Informationen, Energie und/oder Substanzen aufweist.

23. Sonde nach einem der Ansprüche 1 bis 22, **dadurch gekennzeichnet, daß** sie als Implantat ausgeführt ist und eine Kapselwand (14) aufweist, die mit Langzeit-biokompatiblem und sterilisierbarem Material ausgeführt ist.

24. Sonde nach einem der Ansprüche 1 bis 23, **dadurch gekennzeichnet, daß** sie als Implantat eine voll umschließende transparente Kapselwand (14) aufweist, die vorzugsweise aus Glas besteht.

25. Sonde nach einem der Ansprüche 1 bis 24, **dadurch gekennzeichnet, daß** in der Kapsel (12) zumindest ein Therapielicht abstrahlendes Element (32) oder Elementarray angeordnet ist, das Licht zur photodynamischen Therapie hauptsächlich in dem Wellenlängenbereich abstrahlt, in dem das Absorptionsmaximum eines in den Körper eingebrachten Photosensitizers liegt.

26. Sonde nach Anspruch 25, **dadurch gekennzeichnet, daß** das zumindest eine Therapielicht abstrahlende Element (32) bzw. Elementarray in der Kapsel (12) so angeordnet ist, daß der gesamte Raumwinkel homogen ausgeleuchtet wird.

27. Sonde nach Anspruch 25 oder 26, **dadurch gekennzeichnet, daß** das zumindest eine lichtabstrahlende Element (16), das zumindest eine lichtempfangende Element (18) und das zumindest eine Therapielicht abstrahlende Element (32) so ausgerichtet sind, daß lokal in dem Raumwinkel Therapielicht abgestrahlt werden kann, in dem ein Fluoreszenzsignal von dem zumindest einen lichtempfangenden Element (18) empfangen wird, das von dem durch das zumindest eine lichtabstrahlende Element (16) abgestrahlten Anregungslicht hervorgerufen ist.

28. Sonde nach einem der Ansprüche 25 bis 27, **dadurch gekennzeichnet, daß** das zumindest eine Therapielicht abstrahlende Element (32) als Leuchtdiode mit einer Wellenlänge im Bereich von 590 bis 650 nm ausgebildet ist.

## Claims

1. An intracorporeal probe for examination and/or therapy, for example of hollow organs or natural or artificially created body cavities in the human or animal body, the probe (10) being in the form of a capsule (12) which can be introduced into the body without external connection elements, and the probe (10) having at least one light-emitting element (16) and at least one light-receiving element (18), wherein the light-receiving element (18) receives light in another wavelength range than that in which the light-emitting element (16) emits light, **characterized in that** a position-detecting element is arranged in the capsule (12), a position of which can be determined from outside the body, in order to detect the position of the probe (10) and the orientation of the probe (10) with respect to its own axes relative to the hollow organ or body cavity.

2. The probe of claim 1, **characterized in that** the light of the at least one light-emitting element (16) has a shorter wavelength than the light which can be received by the at least one light-receiving element (18).

3. The probe of claim 1 or 2, **characterized in that** the at least one light-emitting element (16) has an emission characteristic covering the entire solid angle.

4. The probe of anyone of claims 1 through 3, **characterized in that** a plurality of light-emitting elements (16) are arranged in the capsule (12) in such a way that the light emission covers the entire solid angle.

5. The probe of anyone of claims 1 through 4, **characterized in that** the at least one light-emitting element (16) is a light-emitting diode.

6. The probe of claim 5, **characterized in that** the light-emitting diode emits in the blue frequency range.

7. The probe of anyone of claims 1 through 6, **characterized in that** the at least one light-receiving element (18) is designed in such a way that it receives light from the entire solid angle range.

8. The probe of anyone of claims 1 through 7, **characterized in that** a plurality of light-receiving elements (18) are arranged in the capsule (12) in such a way that light can be received from the entire solid angle range.

9. The probe of anyone of claims 1 through 8, **characterized in that** an optical filter element is arranged on the at least one light-emitting element (16) and/or on the at least one light-receiving element (18).

10. The probe of anyone of claims 1 through 9, **characterized in that** the capsule (12) contains at least one light-receiving element (20) in the form of an image sensor, in particular of a two-dimensional image sensor, for the purpose of receiving a visual image.

11. The probe of claim 10, **characterized in that** the capsule (12) contains at least one light-emitting element (22) which emits white light.

12. The probe of anyone of claims 1 through 11, **characterized in that** the capsule (12) contains a transmitter element (26) for the purpose of emitting signals from the probe (10) to outside the body.

13. The probe of claim 12, **characterized in that** the capsule contains a signal-preprocessing element (28) which forwards the opto-electrical signal originating from the at least one light-receiving element (18) to the transmitter element.

14. The probe of anyone of claims 1 through 13, **characterized in that** the capsule (12) contains a signal storage element for the purpose of storing signals of the at least one light-receiving element (18).

15. The probe of anyone of claims 1 through 14, **characterized in that** the position-detecting element is designed as a coil system whose position can be detected via an external magnetic field detector.

16. The probe of anyone of claims 1 through 15, **characterized in that** the capsule (12) contains a positioning element which can be controlled from outside the body in order to position the probe.

17. The probe of anyone of claims 1 through 16, **characterized in that** the capsule contains an energy supply element (30) or an element for receiving electromagnetic energy irradiated from outside the body.

18. The probe of anyone of claims 1 through 17, **characterized in that** fluorescent/luminescent marker substances are applied on the capsule (12).

19. The probe of anyone of claims 1 through 18, **characterized in that** the capsule (12) contains at least one luminescent substance which can be excited by excitation from outside the body and emits light through a capsule wall (14).

20. The probe of anyone of claims 1 through 19, **characterized in that** the capsule contains a reservoir for therapeutic substances and/or diagnostic substances which are dispensed inside the body by the probe.

21. The probe of anyone of claims 1 through 20, **characterized in that** an ultrasound transmitter/receiver element for ultrasound imaging is arranged in the probe.

22. The probe of anyone of claims 1 through 21, **characterized in that** it has at least one line leading to outside the body for the purpose of exchanging information, energy and/or substances.

23. The probe of anyone of claims 1 through 22, **characterized in that** it is designed as an implant and has a capsule wall (14) formed with long-term biocompatible and sterilizable material.

24. The probe of anyone of claims 1 through 23, **characterized in that**, as an implant, it has a fully enclosing transparent capsule wall (14), preferably made of glass.

25. The probe of anyone of claims 1 through 24, **characterized in that** the capsule (12) contains at least one element (32) or element array emitting therapeutic light, which element (32) or element array emits light for photodynamic therapy principally in the wavelength range in which the absorption peak of a photosensitizer introduced into the body lies.

26. The probe of claim 25, **characterized in that** the at least one element (32) or element array emitting therapeutic light is arranged in the capsule (12) in such a way that the whole solid angle is illuminated homogeneously.

27. The probe of claim 25 or 26, **characterized in that** the at least one light-emitting element (16), the at least one light-receiving element (18) and the at least one element (32) emitting therapeutic light are oriented in such a way that therapeutic light can be emitted locally in the solid angle in which a fluorescence signal is received by the at least one light-receiving element (18), which signal is produced by the exciting light emitted by the at least one light-emitting element (16).

28. The probe of anyone of claims 25 through 27, **characterized in that** the at least one element (32) emitting therapeutic light is designed as a light-emitting diode with a wavelength in the range of 590 to 650 nm.

## Revendications

1. Sonde intracorporelle pour l'analyse et/ou la thérapie, par exemple, d'organes creux ou de cavités organiques naturelles ou artificielles dans le corps humain ou animal, la sonde (10) étant réalisée en forme de capsule (12) destinée à être introduite dans le corps sans éléments de liaison externes et la sonde (10) comportant au moins un élément émetteur de lumière (16) et au moins un élément récepteur de lumière (18), l'élément récepteur de lumière (18) captant la lumière dans un domaine de longueur d'onde différent de celui de l'élément émetteur de lumière (16), **caractérisée en ce que** dans la capsule (12) est disposé un élément détecteur d'orientation, dont l'orientation peut être déterminée du côté extracorporel en vue de détecter par rapport à l'organe creux ou à la cavité organique la position de la sonde (10) et l'orientation de la sonde (10) par rapport à ses propres axes.

2. Sonde selon la revendication 1, **caractérisée en ce que** la lumière dudit au moins un élément émetteur de lumière (16) a une longueur d'onde plus courte que la lumière qui peut être captée par ledit au moins un élément récepteur de lumière (18).

3. Sonde selon la revendication 1 ou 2, **caractérisée en ce que** ledit au moins un élément émetteur de lumière (16) a une caractéristique de rayonnement couvrant la totalité de l'angle solide.

4. Sonde selon l'une des revendications 1 à 3, **caractérisée en ce qu'**une pluralité d'éléments émetteurs de lumière (16) sont disposés dans la capsule (12) de telle sorte que le rayonnement de la lumière couvre la totalité de l'angle solide.

5. Sonde selon l'une des revendications 1 à 4, **caractérisée en ce que** ledit au moins un élément émetteur de lumière (16) est une diode luminescente.

6. Sonde selon la revendication 5, **caractérisée en ce que** la diode luminescente émet dans le domaine de fréquence du bleu.

7. Sonde selon l'une des revendications 1 à 6, **caractérisée en ce que** ledit au moins un élément récepteur de lumière (18) est conçu de telle sorte qu'il capte la lumière de la totalité du domaine de l'angle solide.

8. Sonde selon l'une des revendications 1 à 7, **caractérisée en ce qu'**une pluralité d'éléments récepteurs de lumière (18) sont disposés dans la capsule (12) de manière à pouvoir capter la lumière dans la totalité du domaine de l'angle solide.

9. Sonde selon l'une des revendications 1 à 8, **caractérisée en ce qu'**un filtre optique est disposé au niveau dudit au moins un élément émetteur de lumière (16) et/ou au niveau dudit au moins un élément récepteur de lumière (18).

10. Sonde selon l'une des revendications 1 à 9, **caractérisée en ce qu'**au moins un élément récepteur de lumière (20) en forme de capteur d'images, en particulier un capteur d'images bidimensionnel, est prévu dans la capsule (12) en vue de capter une image visuelle.

11. Sonde selon la revendication 10, **caractérisée en ce qu'**au moins un élément émetteur de lumière (22), émettant de la lumière blanche, est disposé dans la capsule (12).

12. Sonde selon l'une des revendications 1 à 11, **caractérisée en ce que** dans la capsule (12) est disposé un émetteur (26) destiné à émettre des signaux à partir de la sonde (10) vers le côté extracorporel.

13. Sonde selon la revendication 12, **caractérisée en ce que** dans la capsule est prévu un élément de pré-traitement du signal (28), **en ce que** le signal opto-électrique émanant dudit au moins un élément récepteur de lumière (18) est transmis vers l'émetteur.

14. Sonde selon l'une des revendications 1 à 13, **caractérisée en ce que** dans la capsule (12) est disposée une mémoire de signaux destinée à stocker des signaux dudit au moins un élément récepteur de lumière (18).

15. Sonde selon l'une des revendications 1 à 14, **caractérisée en ce que** le détecteur d'orientation est réalisé sous forme de système à bobines dont la position peut être détectée par l'intermédiaire d'un détecteur de champ magnétique externe.

16. Sonde selon l'une des revendications 1 à 15, **caractérisée en ce que** dans la capsule (12) est disposé un élément de positionnement qui peut être commandé du côté extracorporel en vue de positionner la sonde.

17. Sonde selon l'une des revendications 1 à 16, **caractérisée en ce que** dans la capsule est disposé un élément d'alimentation en énergie (30) ou un élément destiné à recevoir l'énergie électromagnétique injectée du côté extracorporel.

18. Sonde selon l'une des revendications 1 à 17, **caractérisée en ce que** des substances de marquage fluorescentes/luminophores sont déposées sur la capsule (12).

19. Sonde selon l'une des revendications 1 à 18, **caractérisée en ce que** dans la capsule (12) est prévue au moins une substance luminophore qui peut être stimulée par une excitation du côté extracorporel et qui émet à travers une paroi (14) de la capsule.

20. Sonde selon l'une des revendications 1 à 19, **caractérisée en ce que** dans la capsule est prévu un réservoir pour des substances thérapeutiques et/ou des substances de diagnostic qui sont distribuées par la sonde du côté intracorporel.

21. Sonde selon l'une des revendications 1 à 20, **caractérisée en ce qu'**un émetteur d'ultrasons/récepteur d'ultrasons pour la transmission d'images ultrasonores est disposé dans la sonde.

22. Sonde selon l'une quelconque des revendications 1 à 21, **caractérisée en ce qu'**elle comporte au moins une ligne vers le côté extracorporel pour un échange d'informations, d'énergie et/ou de substances.

23. Sonde selon l'une des revendications 1 à 22, **caractérisée en ce qu'**elle est réalisée sous forme d'implant et comporte une paroi de capsule (14) qui est réalisée dans un matériau biocompatible à long terme et apte à être stérilisé.

24. Sonde selon l'une des revendications 1 à 23, **caractérisée en ce que** ladite sonde, sous la forme d'implant, comporte une paroi de capsule (14) transparente enveloppante, qui est réalisée de préférence en verre.

25. Sonde selon l'une des revendications 1 à 24, **caractérisée en ce que** dans la capsule (12) est disposé au moins un élément émetteur de lumière thérapeutique (32) ou une rangée d'éléments émetteurs, qui émettent une lumière pour la thérapie photodynamique essentiellement dans le domaine de longueur d'onde dans lequel se situe un maximum d'absorption d'un agent photosensibilisant injecté dans le corps.

26. Sonde selon la revendication 25, **caractérisée en ce que** ledit au moins un élément émetteur de lumière thérapeutique (32) ou la rangée d'éléments émetteurs sont disposés dans la capsule (12) de manière à éclairer de façon homogène la totalité de l'angle solide.

27. Sonde selon la revendication 25 ou 26, **caractérisée en ce que** ledit au moins un élément émetteur de lumière (16), ledit au moins un élément récepteur de lumière (18) et ledit au moins un élément émetteur de lumière thérapeutique (32) sont orientés de telle sorte que localement la lumière thérapeutique peut être émise dans l'angle solide dans lequel est capté un signal de fluorescence par ledit au moins élément récepteur de lumière (18), qui est suscité par la lumière d'excitation émise par ledit au moins un élément émetteur de lumière (16).

28. Sonde selon l'une des revendications 25 à 27, **caractérisée en ce que** ledit au moins un élément émetteur de lumière thérapeutique (32) est réalisé sous forme de diode luminescente avec une longueur d'onde dans le domaine de 590 à 650 nm.
